# FASCICULE DE BREVET EUROPEEN

(11) **EP 3 174 603 B1**
(45) Date de publication et mention de la délivrance du brevet: **27.11.2019**
(21) Numéro de dépôt: 14759244.8
(22) Date de dépôt: 01.08.2014
(51) Int. Cl.: A61Q 19/00, A61K 8/97, A61K 8/73, A61K 8/99, C11B 1/10

(54) **EXTRAIT DE CAMELLIA JAPONICA ET COMPOSITIONS COSMÉTIQUES LE COMPRENANT**
EXTRAKT VON CAMELLIA JAPONICA UND KOSMETISCHE ZUSAMMENSETZUNGEN DARAUS
CAMELLIA JAPONICA EXTRACT AND COSMETIC COMPOSITIONS THEREOF

(43) Date de publication de la demande: 07.06.2017
(73) Titulaire: Chanel Parfums Beauté, 92200 Neuilly-sur-Seine (FR)
(72) Inventeur: ROSSIGNOL-CASTERA, Anne, F-34160 Restinclieres (FR); L'HERMITTE, Annabelle, F-34000 Montpellier (FR); GENDRONNEAU, Gaëlle, F-77390 Chaumes-en-Brie (FR); HOLDERITH, Serge, F-94300 Vincennes (FR); MAESTRO, Yannick, F-06370 Mouans Sartoux (FR)
(74) Mandataire: Plasseraud IP
(86) Numéro de dépôt international: PCT/FR2014/052013
(87) Numéro de publication internationale: WO 2016/016515

(56) Documents cités:
- FR-A1- 2 943 684
- KR-A- 20090 107 128
- US-A1- 2012 282 244
- US-A1- 2013 146 481

## Description

La présente invention a pour objet un extrait de fleur de *Camellia japonica,* caractérisé en ce qu'il est susceptible d'être obtenu par extraction des fleurs à l'aide d'au moins un corps gras, ainsi que son utilisation en cosmétique, pour hydrater et/ou protéger la peau humaine vis-à-vis du dessèchement.

La peau est principalement constituée de trois couches, à savoir, en partant de la plus superficielle, l'épiderme, le derme et l'hypoderme.

L'épiderme est en particulier constitué de kératinocytes (majoritaires), de mélanocytes (intervenant dans la pigmentation de la peau) et de cellules de Langerhans. Sa fonction est de protéger le corps de l'environnement extérieur et d'assurer son intégrité, et notamment de freiner la pénétration de micro-organismes ou de substances chimiques, et d'empêcher l'évaporation de l'eau contenue dans la peau.

Pour ce faire, les kératinocytes subissent un processus de maturation orientée continu au cours duquel les kératinocytes situés dans la couche basale de l'épiderme forment, au stade terminal de leur différenciation, des cornéocytes qui sont des cellules mortes totalement kératinisées sous forme d'enveloppes cornées constituées de protéines et de lipides tels que des céramides. Lors de ce processus de différenciation, des lipides épidermiques intercornéocytaires sont en outre formés puis organisés sous forme de bicouches (feuillets) dans le *stratum corneum.* Ils participent, avec les enveloppes cornées précitées, à la fonction barrière de l'épiderme.

La fonction barrière de l'épiderme peut toutefois se trouver perturbée dans certaines conditions climatiques (sous l'effet du froid et/ou du vent, par exemple), ou encore sous l'effet du stress ou de la fatigue, notamment, favorisant ainsi la pénétration d'allergènes, d'agents irritants ou de micro-organismes qui occasionnent ainsi un dessèchement cutané susceptible de générer des sensations d'inconfort telles que des tiraillements ou des rougeurs, et également d'altérer l'éclat du teint et la souplesse de la peau.

Pour prévenir ce phénomène ou le corriger, il est connu d'appliquer sur la peau des compositions cosmétiques renfermant des agents hygroscopiques, tels que des sucres ou des polyols, destinés à capter l'eau présente dans la peau et à freiner ainsi son évaporation. Par ailleurs, ces compositions incorporent fréquemment des actifs agissant sur une ou plusieurs des différentes cibles biologiques intervenant soit dans les processus de régénération de la peau, en particulier dans la différenciation des kératinocytes, la synthèse des lipides épidermiques et la cohésion des cornéocytes, soit dans la synthèse endogène de constituants du facteur d'hydratation naturel (NMF, Natural Moisturizing Factor) de la peau, en particulier dans la synthèse de protéoglycanes.

Des exemples de tels actifs sont notamment les α- et β-hydroxyacides, notamment l'acide lactique, l'acide glycolique et l'acide salicylique, l'urée, ou les composés aminosulfoniques.

Toutefois, il subsiste toujours le besoin de proposer de nouveaux actifs cosmétiques permettant une hydratation plus efficace de la peau.

En outre, compte tenu de la recherche toujours croissante par les consommateurs de produits naturels renfermant le moins d'ingrédients synthétiques possibles, et des contraintes réglementaires de plus en plus lourdes pesant sur les composés issus de l'industrie chimique, il serait souhaitable que ces actifs cosmétiques soient d'origine végétale.

La Demanderesse a déjà montré, dans sa demande internationale WO2011/083110 qu'il était possible de lutter efficacement contre le dessèchement de la peau, en utilisant un extrait hydroalcoolique de Camellia, ce dernier agissant par stimulation de l'expression de l'ARNm de HSP32, stimulation de l'expression de la protéine HSP27 et stimulation de l'expression de la protéine PPAR-β/δ sur les kératinocytes traités. La Demanderesse a également décrit un procédé d'extraction dans sa demande internationale WO2010/112760.

Toutefois, les extraits du type de ceux décrits dans la demande WO2011/083110 étant véhiculés dans un milieu hydroalcoolique, ils ne conviennent pas à toutes les applications cosmétiques, notamment lorsque les compositions souhaitées sont anhydres ou présentent une phase grasse continue. De plus, certains alcools, tels que l'éthanol ou le méthanol, pour la préparation de l'extrait peuvent s'avérer desséchants pour la peau, ce qui va à l'inverse de l'effet initialement recherché.

Des extraits huileux de Camellia ont déjà été proposés tels que l'huile de Camélia raffinée commercialisée par la société ARDEX produite à partir de la variété Camellia oleifera. Toutefois, ces extraits huileux, bien qu'efficaces pour éviter le desséchement de la peau généré par l'utilisation d'alcools, ne montrent étonnamment aucun effet d'amélioration de l'hydratation de la peau.

Il est ainsi du mérite de la demanderesse d'avoir mis au point un extrait huileux de fleur de *Camellia japonica* ne présentant pas les inconvénients des extraits hydroalcooliques actuellement sur le marché, tout en préservant, voire en améliorant, leur action hydratante pour la peau par stimulation de l'expression du gène KRT2 (Kératine 2).

La présente invention a ainsi pour objet, selon un premier aspect, un extrait de fleur de *Camellia japonica* susceptible d'être obtenu au moyen d'un procédé d'extraction comprenant les étapes suivantes :
a) le mélange et l'imprégnation d'une poudre de fleurs de *Camellia japonica* avec un corps gras ou un mélange de corps gras à une température supérieure à la température de fusion dudit corps gras ou dudit mélange et sous atmosphère dépourvue ou essentiellement dépourvue d'oxygène,
b) la micro-dispersion de la poudre de fleurs de *Camellia japonica* dans le corps gras ou ledit mélange de corps gras à une température supérieure à la température de fusion dudit corps gras ou dudit mélange, sous atmosphère dépourvue ou essentiellement dépourvue d'oxygène,
c) le chauffage du mélange ainsi obtenu à une température comprise entre 60 et 180°C pendant une durée comprise entre 1 et 10 minutes, sous atmosphère dépourvue ou essentiellement dépourvue d'oxygène,

l'étape c) pouvant être réalisée avant, pendant ou après l'étape b).

Selon une caractéristique essentielle de l'invention, les étapes a), b) et c) du procédé de préparation de l'extrait huileux de *Camellia japonica* sont conduites en atmosphère dépourvue ou essentiellement dépourvue d'oxygène. Ceci signifie que l'on travaille sous gaz ou atmosphère inerte ou sous vide ou vide partiel. La teneur résiduelle en oxygène doit être suffisamment basse pour ne pas provoquer de réactions d'oxydation sensibles à la température du traitement thermique. On peut donc conduire ces étapes sous atmosphère inerte, par exemple sous azote et de préférence sous balayage continu d'azote, permettant l'extraction de l'oxygène présent ou susceptible de se former. On peut employer un réacteur clos avec une extraction continue de l'oxygène par flux d'azote. On peut aussi faire un barbotage d'azote, associé au flux d'azote, au moins au début du traitement thermique. On peut aussi conduire ces étapes sous vide. Procéder ainsi confère un avantage supplémentaire, à savoir l'entraînement des matières volatiles avec un effet de désodorisation du mélange.

L'invention a également pour objet, selon un deuxième aspect, une composition cosmétique comprenant, dans un milieu physiologiquement acceptable, au moins un extrait de fleur de *Camellia japonica* tel que décrit précédemment.

Enfin, la présente invention a pour objet, selon un troisième aspect, l'utilisation cosmétique dudit extrait ou de ladite composition pour hydrater la peau humaine.

L'extrait de Camellia mis en œuvre dans le cadre de la présente invention présente l'avantage d'être un extrait lipidique, et en particulier un extrait huileux, ce qui lui procure un effet hydratant de la peau amélioré.

Dans le cadre de la présente invention, l'extrait de fleur de *Camellia japonica* est susceptible d'être obtenu au moyen d'un procédé d'extraction comprenant une étape a) de mélange et d'imprégnation d'une poudre de fleurs de *Camellia japonica* avec un corps gras ou un mélange de corps gras à une température supérieure à la température de fusion dudit corps gras et sous atmosphère dépourvue ou essentiellement dépourvue d'oxygène.

Le Camellia est un genre de plantes à fleurs de la famille des Théacées, originaire d'Asie orientale et méridionale de l'est depuis la chaîne de l'Himalaya jusqu'au Japon et en Indonésie. Les fleurs de Camellia sont reconnues entre autres pour leurs propriétés antibactériennes, anti-oxydantes, anti-inflammatoires, astringentes et toniques. Le nombre d'espèces que contient le genre diffère suivant les botanistes, et varie entre 100 à 250 espèces. On peut notamment citer les fleurs de couleur blanche, notamment la variété *Alba plena,* des fleurs de *Camellia japonica* de couleur rouge très foncé du type rouge chocolat à rouge noir, notamment les variétés *Black magic, Kuro tsubaki,* Black Domino, Koronkoku, Kon wabisuke, Kuro wabisuke , Murasaki-no-ue, Sir Victor Davis, des fleurs de camellias hybrides de Kuro Tsubaki dont les noms de variété sont Night Rider ou Black Opal. Les variétés Black Magic et Night Rider sont les variétés de *Camellia japonica* les plus courantes en France. Les couleurs des fleurs de camélia peuvent varier en fonction du pH, des métaux et des métalloïdes présents dans le sol ou substrat. Les camellias fleurissent généralement de mi-février à avril. Il est toutefois possible d'obtenir des floraisons dès octobre par traitement hormonal.

L'extrait selon l'invention est obtenu à partir des fleurs de *Camellia japonica,* et de préférence, à partir des fleurs de couleur blanche, notamment de la variété *Alba plena.* De préférence, les fleurs de *Camellia japonica* utilisées dans l'invention sont cultivées en France.

L'extrait de fleurs de *Camellia japonica* se présente de préférence sous forme de poudre dispersible. Par dispersible, on entend que la poudre de fleurs de *Camellia japonica* se présente sous une forme dissociée apte à être dispersée finement, et par exemple, la matière première est sous forme particulaire et de préférence pulvérulente. Les fleurs de *Camellia japonica* fraiches sont par exemple dans un premier temps isolées des tiges puis ouvertes et mises à plat sur des claies. Elles sont ensuite déshydratées en conditions douces, soit à température ambiante à l'abri de la lumière soit dans un séchoir ventilé à une température inférieure à 35°C. Les fleurs sont de préférence séchées jusqu'à obtention d'une teneur en matière sèche supérieure à 80% et préférentiellement supérieure à 85%.

Les fleurs sont ensuite réduites en poudre dispersible par tout procédé de broyage classiquement connu de l'homme du métier, par exemple à température ambiante dans un broyeur à couteaux ou, selon un mode de réalisation préféré, par broyage à basse température. Pour un broyage à basse température, les fleurs sont de préférence refroidies à -80°C et immédiatement broyées dans un mixer à hélices à une température comprise entre -20 et -80°C, pour obtenir une poudre fine et régulière. La cryogénisation permet avantageusement d'assurer une meilleure conservation des propriétés hydratantes des molécules contenues dans les fleurs.

De préférence, la poudre dispersible de fleurs de *Camellia japonica* mise en œuvre pour la préparation de l'extrait selon l'invention présente une taille moyenne de particules inférieure à 500 pm, préférentiellement inférieure à 300 µm. La poudre de fleurs de *Camellia japonica* présente une odeur florale douce et une couleur allant de blanc crème à rouge brun.

Le ou les corps gras mis en œuvre dans le procédé de préparation de l'extrait de *Camellia japonica* selon l'invention sont de préférence naturels ou d'origine naturelle. Parmi les corps gras naturels, on peut employer notamment des huiles végétales raffinées comprenant moins de 0,1 % d'eau en poids ou des huiles végétales vierges ou non raffinées pouvant contenir de 0,1 à 2 % d'eau en poids, de préférence de 0,1 à 0,3 % d'eau en poids.

En particulier, le corps gras utilisé comme solvant d'extraction est préférentiellement d'origine végétale, et peut être une huile végétale liquide à température ambiante (20-25°C), un beurre végétal présentant un point de fusion compris entre 25 et 40°C, ou une cire végétale présentant un point de fusion supérieur à 40°C. Selon un mode préféré de réalisation, le corps gras utilisé comme solvant d'extraction est une huile végétale liquide à une température inférieure à la température ambiante, et en particulier liquide à une température d'environ 20°C.

A titre d'exemple d'huiles pouvant être utilisées pour l'obtention de l'extrait selon l'invention, on peut citer l'huile de camélia, l'huile de colza, l'huile de tournesol, l'huile d'olive, l'huile de sésame, l'huile de noyaux d'abricot, l'huile de pépin de raisin, l'huile d'amande douce, l'huile de carthame, l'huile de noisette, l'huile d'argan, l'huile de rosier muscat, l'huile d'onagre, l'huile de bourrache, la cire liquide de jojoba, et leurs mélanges. Il sera choisi préférentiellement une huile source d'acides gras polyinsaturés oméga 6 ou oméga 3 pouvant jouer un rôle positif sur la fluidité membranaire et sur l'hydratation de la peau.

Selon un mode de réalisation de l'invention, le ratio entre la poudre de fleurs de *Camellia japonica* et le corps gras dans l'étape a) est compris entre 1:0,5 et 1:20, de préférence entre 1:19 et 1:1, et plus préférentiellement entre 1:9 et 1:3, ratio exprimé en masse/masse de corps gras ou en masse/volume de corps gras.

L'étape a) est conduite à une température supérieure ou égale au point de fusion du corps gras ou du mélange de corps gras utilisé. En particulier, la température est avantageusement comprise entre cette température de fusion et la température de fusion +20°C, de préférence +10°C. La température ambiante (20-25°C) est parfaitement adaptée pour les corps gras tels que les huiles liquides à cette température. La durée de l'étape a) peut être comprise entre 1 et 48 heures, de préférence entre 5 et 40 heures, plus préférentiellement entre 12 et 36 heures, encore plus préférentiellement entre 20 et 30 heures, et selon un mode particulièrement préféré de réalisation, la durée de l'étape a) d'imprégnation est d'environ 24 heures.

L'étape a) est réalisée sous atmosphère dépourvue ou essentiellement dépourvue d'oxygène, et de préférence sous atmosphère saturée d'azote.

Le procédé permettant d'obtenir l'extrait selon l'invention comprend également une étape b) de micro-dispersion de la poudre de fleurs de *Camellia japonica* dans le corps gras à une température supérieure à la température de fusion dudit corps, sous atmosphère dépourvue ou essentiellement dépourvue d'oxygène.

Cette étape b) permet non seulement la micro-dispersion de la poudre de Camellia à extraire, mais également, sur des tissus cellulaires, la rupture des cellules, ce qui favorise la dispersion des molécules extraites dans le corps gras naturel. Cet effet peut être obtenu par traitement du mélange par cavitation ultrasonore. La cavitation et la dispersion sous ondes ultrasonores sont de préférence réalisées dans un réacteur fermé équipé d'un générateur d'ultrasons à basse fréquence de cavitation, notamment inférieure à 100 kHz et de préférence de l'ordre de 20 à 30 kHz.

La durée du traitement sous ultrasons est notamment comprise entre 2 et 30 minutes, de préférence entre 10 et 20 minutes.

L'étape b) est avantageusement conduite à température ambiante ou à une température supérieure au point de fusion du corps gras ou des corps gras utilisés. La température est avantageusement comprise entre cette température de fusion et la température de fusion +20°C, de préférence +10°C. La température ambiante (20-25°C) est parfaitement adaptée pour les huiles liquides à cette température.

Le procédé d'obtention de l'extrait de fleur de Camellia selon l'invention comprend également une étape c) de chauffage du mélange obtenu à l'étape a) ou b) de ladite poudre de fleurs de *Camellia japonica* avec le ou lesdits corps gras à une température comprise entre 80 et 180°C pendant une durée comprise entre 1 et 10 minutes, sous atmosphère dépourvue ou essentiellement dépourvue d'oxygène.

Dans un mode de réalisation préféré, la température de l'étape c) est comprise entre 100 et 150°C, de préférence entre 110 et 130°C.

L'étape c) de chauffage est mise en œuvre pendant une très courte durée allant de 1 à 10 minutes, de préférence de 1 à 5 minutes, et plus préférentiellement de 1 à 3 minutes, cette durée correspondant au temps de maintien de la température de traitement une fois cette température atteinte. Le temps de montée en température est également très court, notamment inférieur ou égal à 5 minutes, de préférence de 1 à 5 min, et plus préférentiellement de 1 à 3 minutes.

Tout système de chauffage thermique rapide peut être utilisé, et dans un mode préféré de réalisation, le traitement thermique est assuré par des micro-ondes. L'utilisation d'une source micro-ondes dans un réacteur fermé permet d'atteindre en un temps très court les températures souhaitées. Le chauffage à haute température permet par ailleurs d'accroître le pouvoir de solubilisation du corps gras utilisé et favorise le contact entre la poudre de fleur de Camellia et ledit corps gras, favorisant ainsi le rendement de l'extraction. Selon un mode préféré de réalisation, le générateur de micro-ondes mis en œuvre pour le chauffage de l'étape c) présente puissance utile allant de 500 à 10000 Watts par kilogramme de mélange, de préférence de l'ordre de 700 à 1500 Watts par kilogramme de mélange, et plus préférentiellement de l'ordre de 1000 Watts par kilogramme de mélange.

Suivant une caractéristique avantageuse, on ajoute lors de l'étape c) ou juste avant, un composé piégeur ou réducteur d'oxygène. Il est possible ainsi d'ajouter de la vitamine C, sous forme d'acide ascorbique pur, de sel tel que l'ascorbate de sodium ou de palmitate d'ascorbyle, de l'acide citrique ou de l'acide lactique sous forme libre ou ester ou des lécithines, ou encore une combinaison de ces composés. Il sera ajouté une quantité individuelle de 0,01 à 1 % en poids dans le mélange, préférentiellement de 0,1 à 0,5 % en poids dans le mélange.

Les étapes a), b) et/ou c) sont avantageusement conduites en l'absence de lumière ou de tout rayonnement oxydant tels que les UV pour limiter les risques de photo-oxydation et de dégradation des molécules photosensibles.

Les étapes a), b) et/ou c) peuvent être réalisées avec ou sans agitation du mélange et préférentiellement avec agitation.

Selon un mode de réalisation le procédé consiste en une séquence combinée des étapes a), b) et c), l'ordre des étapes b) et c) étant indifférent, chaque étape étant réalisée au minimum une fois chacune.

Les étapes b) et c) peuvent en particulier être conduites en simultané. Selon un mode de réalisation préféré, les étapes b) et c) sont réalisées au moins une seconde fois et par exemple n fois ; elles correspondent alors aux étapes bn) et cn), n correspondant au nombre total de répétitions du cycle {(étape a) + (étape b)}, n étant au moins égal à 2, de préférence n étant égal à 2.

Dans un mode de réalisation, il peut être réalisé, entre chaque étape, ou après la dernière étape, une période de diffusion passive des composés extraits dans l'huile et de refroidissement éventuellement sous agitation douce en système fermé sous atmosphère dépourvue ou essentiellement dépourvue d'oxygène.

La durée de cette étape doit être suffisante pour une bonne diffusion des actifs dans l'huile. Cette durée peut être notamment comprise entre 1 h et 12 h, de préférence entre 1 h et 5 h. Le refroidissement peut être réalisé de toute manière connue, notamment par refroidissement passif ou à l'aide de moyens de refroidissement. Cette étape de refroidissement est avantageusement conduite en atmosphère dépourvue ou essentiellement dépourvue d'oxygène, comme les étapes a) b) et c). Elle est avantageusement conduite en l'absence de lumière ou de tout rayonnement oxydant tels que les UV.

A titre de finition, le procédé peut comprendre une ou plusieurs étape(s) de clarification de l'extrait huileux. On entend par clarification toutes les séparations mécaniques connues de l'homme du métier. Elles peuvent par exemple être choisies parmi la filtration, la décantation, la centrifugation, l'essorage, ou une association de ces techniques. De préférence, la séparation est effectuée par centrifugation ou filtration. Cette étape de séparation peut être réalisée à une température comprise entre 40 et 60°C dans une essoreuse munie d'une toile filtrante de porosité inférieure à 10 µm et préférentiellement inférieure à 5 µm et à une vitesse supérieure à 2500 tours/min.

Les étapes de clarification permettent l'obtention d'un produit à la fois substantiellement limpide à l'œil et exempt de microparticules en suspension.

Selon un mode préféré de réalisation, l'extrait de *Camellia japonica* selon la présente invention est susceptible d'être obtenu au moyen du procédé précédemment décrit, suivant les étapes a), bn), optionnellement une étape de diffusion/refroidissement, cn), optionnellement une autre étape de diffusion/refroidissement, a), et une étape finale de séparation.

L'extrait de fleur de *Camellia japonica* susceptible d'être obtenu au moyen du procédé selon l'invention est concentré en actifs et peut se présenter sous forme de solution huileuse, de micro-dispersion huileuse, de micro-suspension huileuse ou de microémulsion huileuse, forme qui soit stable dans le temps. En particulier, l'extrait selon l'invention se présente sous la forme d'une huile transparente à l'œil, brillante, homogène, de couleur jaune clair à jaune orangé.

L'extrait de fleur de *Camellia japonica* est utilisé selon l'invention à des fins cosmétiques, pour hydrater la peau humaine ou la protéger vis-à-vis du dessèchement. Il peut également être utilisé pour lutter contre les signes cutanés résultant d'une fonction barrière perturbée, y compris la rugosité de la peau, les inconforts dont les rougeurs, les tiraillements, les picotements et les démangeaisons, la perte d'éclat du teint ou teint terne, la perte de souplesse de la peau et les gerçures.

L'effet hydratant de la composition utilisée selon l'invention peut notamment être observé par une augmentation de l'expression du gène kératine 2 (KTR2), selon des techniques habituelles bien connues de l'homme du métier.

L'invention a également pour objet une composition cosmétique comprenant un extrait de fleur de *Camellia japonica* tel que décrit précédemment.

De façon préférée, la composition selon l'invention, contenant l'extrait de fleur de *Camellia japonica,* est appliquée sur une peau sèche, de préférence non pathologique. Elle peut être avantageusement appliquée sur la peau du visage, du cou et éventuellement du décolleté ou en variante sur une partie quelconque du corps. La composition renfermant cet extrait peut être appliquée le matin et/ou le soir, sur l'ensemble du visage, du cou et éventuellement du décolleté voire du corps.

La composition mise en œuvre selon l'invention comprend généralement, outre l'extrait décrit précédemment, un milieu physiologiquement acceptable et de préférence cosmétiquement acceptable, c'est-à-dire qui convient à une utilisation en contact avec la peau humaine sans risque de toxicité, d'incompatibilité, d'instabilité, de réponse allergique et notamment qui ne provoque pas de sensations d'inconfort (rougeurs, tiraillements, picotements...) inacceptables pour l'utilisateur.

Ce milieu contient généralement de l'eau et éventuellement d'autres solvants comme des huiles.

La composition utilisée selon l'invention peut se présenter sous toute forme adaptée à une application topique sur la peau et en particulier sous forme de formulation anhydre, d'émulsion huile-dans-eau, eau-dans-huile, éventuellement multiple (E/H/E ou H/E/H), qui peuvent éventuellement être des microémulsions ou des nanoémulsions, ou sous forme de poudre. On préfère que cette composition se présente sous la forme d'une émulsion huile-dans-eau.

Cette composition est de préférence utilisée en tant que produit de soin ou de nettoyage de la peau du visage et/ou du corps et elle peut se présenter notamment sous forme de fluide, de gel ou de mousse, conditionnés par exemple dans un flacon-pompe, un aérosol ou un tube, ou de crème conditionnée par exemple dans un pot. En variante, elle peut avoir la forme d'un produit de maquillage et en particulier d'un fond de teint ou d'une poudre libre ou compacte.

Outre l'extrait de fleur de *Camellia japonica* précédemment décrit, la composition selon l'invention peut également comprendre au moins un additif usuel dans le domaine cosmétique, tel que par exemple au moins un composé choisi parmi un agent gélifiant et/ou épaississant, un agent tensioactif ou co-tensioactif, un corps gras liquide ou une huile, une cire, un élastomère de silicone, un filtre solaire, un colorant, un agent matifiant ou une charge, un pigment, un agent tenseur, un conservateur, un séquestrant, un parfum et leurs mélanges.

Notamment, la composition selon l'invention peut contenir, de manière non limitative, un ou plusieurs des additifs suivants :
- Un ou plusieurs agent(s) gélifiants(s) et/ou épaississant(s) de la phase aqueuse, choisi par exemple parmi les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique tels que les ammonium acryloyldiméthyltaurate/VP copolymer et ammonium acryloyldiméthyl-taurate/beheneth-25 méthacrylate copolymer, notamment ceux vendus sous les dénominations Aristoflex® AVC et HMB de Clariant, ou encore les acrylates/C10-30 Alkyl Acrylate Crosspolymer vendu sous la démonmiation commerciale PEMULEN® TR-1 ou TR-2, Carbopol® 1382, Carbopol® Ultrez 20 par la société Novéon, les dérivés cellulosiques, les gommes d'origine végétale (acacia ou arabique, agar, guar, caroube, alginates, carraghénanes, pectine) ou d'origine microbienne (xanthane, pullulane), les argiles (laponite). Ledit agent gélifiant et/ou épaississant peut être présent dans la composition en une teneur de l'ordre de 0,01 à 5% en poids, par rapport au poids total de la composition ;
- Un ou plusieurs agent(s) tensioactif(s), de préférence émulsionnants, qu'ils soient non ioniques, anioniques, cationiques ou amphotères, et en particulier les esters d'acides gras et de polyols tel que les esters d'acides gras et de glycérol oxyalkylénés (plus particulièrement polyoxyéthylénés), les esters d'acides gras et de sorbitan oxyalkylénés, les esters d'acides gras oxyalkylénés (oxyéthylénés et/ou oxypropylénés) comme le mélange PEG-100 Stearate/Glyceryl Stearate commercialisé par exemple par la société Croda Inc. sous la dénomination Arlacel® 165 et les esters d'acides gras et de sucrose comme le stéarate de sucrose; les éthers d'alcool gras et de sucre, notamment les alkylpolyglucosides (APG) tels que le décylglucoside et le laurylglucoside commercialisés par exemple par la société Henkel sous les dénominations respectives Plantaren® 2000 et Plantaren® 1200, le cétostéarylglucoside éventuellement en mélange avec l'alcool cétostéarylique, commercialisé par exemple sous la dénomination Montanov® 68 par la société Seppic, ainsi que l'arachidyl glucoside, par exemple sous la forme du mélange d'alcools arachidique et béhénique et d'arachidylglucoside commercialisé sous la dénomination Montanov® 202 par la société Seppic ; les éthers d'alcools gras et de polyéthylèneglycol; les polysiloxanes modifiés polyéthers; la bétaïne et ses dérivés; les polyquaterniums; les sels de sulfate d'alcools gras éthoxylés; les sulfosuccinates; les sarcosinates; les alkyl- et dialkylphosphates et leurs sels; et les savons d'acides gras. Ledit agent tensioactif peut être présent dans la composition dans une teneur de l'ordre de 0,1 à 8%, de préférence 0,5 à 3% en poids, par rapport au poids total de la composition ;
- Un ou plusieurs co-tensioactif(s) tels que les alcools gras linéaires à longue chaîne carbonée (C14-C20) et en particulier les alcools cétylique et stéarylique, ledit agent tensioactif étant présent dans la composition à raison de 0,1 à 5%, de préférence 0,5 à 2% en poids, par rapport au poids total de la composition ;
- Un ou plusieurs corps gras liquide(s) à température ambiante, communément dénommé(s) huiles(s), volatile(s) ou non volatile(s), hydrocarboné(s), siliconée(s), linéaire(s), cyclique(s) ou ramifiée(s), par exemple, les huiles de silicone telles que les polydiméthylsiloxanes (diméthicones), les polyalkylcyclosiloxanes (cyclométhicones) et les polyalkylphénylsiloxanes (phenyldiméthicones); les huiles synthétiques telles que les huiles fluorées, les alkyl benzoates et les hydrocarbures ramifiés tels que le polyisobutylène, l'isododécane; les huiles minérales (paraffine); végétales (huile d'amande douce, de macadamia, de pépin de cassis, de jojoba ou encore de camelina sativa telle que l'huile vendue sous la dénomination commerciale Lipex® Omega 3/6 par la société Unipex) ; les alcools gras, les amides grasses, les acides ou les esters gras comme le benzoate d'alcools en C12-C15 vendu sous la dénomination commerciale Finsolv® TN par la société Innospec ou encore l'isononyl isononanoate vendu sous la dénomination commerciale Wickenol® 151 par la société Alzo Inc., le palmitate d'octyle, le lanolate d'isopropyle, les triglycérides dont ceux des acides caprique/caprylique, le dicaprylyl carbonate vendu sous la dénomination Cetiol® CC par la société Cognis ; de préférence à raison de 0,1 à environ 10%, de préférence, de 0,5 à 5% en poids, par rapport au poids total de la composition ;
- Une ou plusieurs cires (composé solide ou substantiellement solide à température ambiante, et dont le point de fusion est généralement supérieur à 35°C), telles que l'ozokérite, la cire de polyéthylène, la cire d'abeille ou la cire de carnauba, de préférence à raison de 0,01 à environ 5%, de préférence 0,5 à 5% en poids, par rapport au poids total de la composition ;
- Un ou plusieurs élastomère(s) de silicone obtenus notamment par réaction, en présence d'un catalyseur, d'un polysiloxane ayant au moins un groupe réactif (hydrogène ou vinyle, notamment) et portant au moins un groupe alkyle (notamment méthyle) ou phényle, terminal et/ou latéral, avec un organosilicone tel qu'un organohydrogénopolysiloxane, de préférence à raison de 0,1 à environ 20%, de préférence 0,25 à 15% en poids, par rapport au poids total de la composition ;
- Un ou plusieurs filtre(s) solaire(s), notamment les filtres organiques, tels que les dérivés de dibenzoylméthane (dont le butyl méthoxydibenzoylméthane vendu en particulier par DSM sous le nom commercial Parsol® 1789), les dérivés d'acide cinnamique (dont l'éthylhexyl méthoxycinnamate vendu en particulier par DSM sous le nom commercial Parsol® MCX), les salicylates, les acides para-aminobenzoïques, les β-β'-diphénylacrylates, les benzophénones, les dérivés de benzylidène camphre, les phénylbenzimidazoles, les triazines, les phénylbenzotriazoles et les dérivés anthraniliques; ou les filtres inorganiques, à base d'oxydes minéraux sous forme de pigments ou de nanopigments, enrobés ou non, et en particulier à base de dioxyde de titane ou d'oxyde de zinc; de préférence à raison de 0,1 à environ 30%, mieux, de 0,5 à 20% en poids, par rapport au poids total de la composition ;

- Un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0,1 à environ 2% en poids, par rapport au poids total de la composition ;
- Une ou plusieurs charges, en particulier des agents matifiants ou des charges à effet flouteur, et en particulier les poudres à effet soft-focus.

Par charge, il faut comprendre des particules incolores ou blanches, minérales ou de synthèse, lamellaires ou non lamellaires, adaptées à donner du corps ou de la rigidité à la composition et/ou de la douceur, de la matité et de l'uniformité immédiate à l'application. Ces charges peuvent notamment modifier voire masquer les rides par un effet de camouflage, ou un effet de floutage.

Les agents matifiants peuvent être choisis parmi les polymères matifiants (en solution, en dispersion ou sous forme de particules) et les particules inorganiques qui réduisent la brillance de la peau et unifient le teint.

L'agent matifiant pourra notamment être choisi parmi un amidon, le talc, des microbilles de cellulose, des fibres végétales, des fibres synthétiques, en particulier de polyamides (les poudres de Nylon® telles que Nylon-12 (Orgasol® commercialisé par la société Atochem), des microsphères de copolymères acryliques notamment de poly(méth)acrylate de méthyle (particules PMMA ou les particules Micropearl® M310 vendue par la société Seppic), les poudres de silice, les poudres de résine de silicone, les poudres de polymères acryliques, les poudres de polyéthylène, les organopolysiloxanes réticulés élastomères (commercialisés notamment sous les dénominations KSG® par la société Shin-Etsu, sous les dénominations Trefil®, BY29® ou EPSX® par la société Dow Corning ou sous les dénominations Gransil® par la société Grant Industries), les poudres composites de talc/dioxyde de titane/alumine/silice, les poudres de silicates, et leurs mélanges.

La charge à effet « soft focus » peut donner de la transparence au teint et un effet flou. De préférence, les charges « soft focus » ont une taille moyenne de particules inférieure ou égale à 30 microns, plus préférentiellement inférieure ou égale à 15 microns. Ces charges « soft focus » peuvent être de toutes formes et en particulier être sphériques ou non sphériques. Elles peuvent être choisies parmi les poudres de silice et silicates, notamment d'alumine, les poudres de type polyméthyl méthacrylate (PMMA ou Micropearl® M310), le talc, les composites silice/Ti02 ou silice/oxyde de zinc, les poudres de polyéthylène, les poudres d'amidon, les poudres de polyamides, les poudres de copolymères styrène/acrylique, les élastomères de silicone, et leurs mélanges.

De préférence ces agents matifiants ou charges à effet soft-focus sont utilisés à raison de 0,1 à environ 10% en poids, par rapport au poids total de la composition, de préférence à raison de 0,1 à environ 7% en poids.
- Une ou plusieurs pigments blancs ou colorés, nacrés ou non, minéraux et/ou organiques, enrobés ou non, insolubles dans le milieu, destinées à colorer et/ou opacifier la composition. Ils peuvent être de taille usuelle ou nanométrique. On peut citer, parmi les pigments minéraux, le dioxyde de titane, éventuellement traité en surface, les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium. Les pigments nacrés ou nacres sont des particules irisées qui réfléchissent la lumière. Ces pigments nacrés peuvent être choisis parmi les pigments nacrés blancs tels que le mica recouvert de titane, ou d'oxychlorure de bismuth, les pigments nacrés colorés tels que le mica titane avec des oxydes de fer. Les pigments peuvent avoir subi un traitement de surface. De préférence, ces pigments sont utilisés à raison de 0,1 à environ 10% en poids, par rapport au poids total de la composition, de préférence à raison de 0,1 à environ 5% en poids.
- Un ou plusieurs agents tenseurs. Par agent tenseur, il faut comprendre un composé adapté à tendre la peau et, par cet effet de tension, lisser la peau et y faire diminuer voire disparaître de façon immédiate les rides et les ridules. Comme agents tenseurs, on peut citer les polymères d'origine naturelle; les silicates mixtes ; les particules colloïdales de charges inorganiques ; les polymères synthétiques ; et leurs mélanges. On peut citer notamment : les polymères d'origine végétale ou microbienne, les polymères issus des phanères, les protéines d'oeuf et les latex d'origine naturelle. Ces polymères sont de préférence hydrophiles. Comme polymères d'origine végétale, on peut citer en particulier les protéines et hydrolysats de protéines, et plus particulièrement les extraits de céréales, de légumineuses et d'oléagineux, tels que les extraits de maïs, de seigle, de froment, de sarrasin, de sésame, d'épeautre, de pois, de tapioca, de fève, de lentille, de soja et de lupin. D'autres agents tenseurs pouvant être mis en œuvre selon l'invention sont les polysaccharides d'origine naturelle, notamment l'amidon issu notamment de riz, de maïs, de tapioca, de pomme de terre, de manioc, de pois ; les carraghénanes, gommes d'acacia (gomme arabique), alginates, agars, gellanes, gommes xanthanes, polymères cellulosiques et pectines, avantageusement en dispersion aqueuse de microparticules de gel, les dérivés cellulosiques, et leurs mélanges. Les polymères synthétiques se présentent généralement sous forme d'un latex ou d'un pseudolatex et peuvent être de type polycondensat ou obtenus par polymérisation radicalaire. On peut citer notamment les dispersions de polyester/polyuréthane et de polyéther/ polyuréthane. De préférence, l'agent tenseur est un copolymère de PVP/dimethiconylacrylate et de polyuréthane hydrophile (Aquamere® S-2011® de la société Hydromer).
- Un ou plusieurs conservateur(s) ;
- Les séquestrants tels que les sels d'EDTA;
- Les parfums;
- et leurs mélanges.

Des exemples de tels adjuvants sont cités notamment dans le Dictionnaire CTFA (International Cosmetic Ingredient Dictionary and Handbook publié par The Cosmetic, Toiletry and Fragrance Association, 11ème Edition, 2006) qui décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

L'homme du métier est en mesure de choisir, parmi l'ensemble de ces éventuels additifs, aussi bien la composition que la quantité de ceux qui seront ajoutés à la composition, de telle sorte que celle-ci conserve l'ensemble de ses propriétés.

En outre, la composition selon la présente invention peut éventuellement contenir divers agents actifs qui peuvent être choisis dans le groupe constitué des vitamines, des antioxydants, des agents hydratants, des agents anti-pollution, les agents kératolytiques, des astringents, des anti-inflammatoires, des agents blanchissants et des agents favorisant la microcirculation. De préférence, la composition comprend au moins un actif choisi parmi les agents hydratants, les antioxydants, les agents favorisant la microcirculation et leurs mélanges.

Des exemples de vitamines incluent les vitamines A, B1, B2, B6, C et E et leurs dérivés, l'acide pantothénique et ses dérivés et la biotine.

Des exemples d'antioxydants incluent l'acide ascorbique et ses dérivés tels que le palmitate d'ascorbyle, le tétraisopalmitate d'ascorbyle, l'ascorbyl glucoside, le magnésium ascorbyl phosphate, le sodium ascorbyl phosphate et le sorbate d'ascorbyle; le tocophérol et ses dérivés, tels que l'acétate de tocophérol, le sorbate de tocophérol et d'autres esters de tocophérol; le BHT et BHA; les esters de l'acide gallique, l'acide phosphorique, l'acide citrique, l'acide maléique, l'acide malonique, l'acide succinique, l'acide fumarique, la céphaline, l'hexamétaphosphate, l'acide phytique, et les extraits de plantes, par exemple de racines de Zingiber Officinale (Gingembre) tel que le Blue Malagasy Ginger commercialisé par la société BIOLANDES, de Chondrus crispus, Rhodiola, Thermus thermophilus, la feuille de maté, le bois de chêne, l'écorce de Rapet Kayu, les feuilles de Sakura et les feuilles d'ylang ylang.

Des exemples d'agents hydratants incluent le polyéthylène glycol, le propylène glycol, le dipropylène glycol, la glycérine, le butylène glycol, le xylitol, le sorbitol, le maltitol, les mucopolysaccharides, tels que l'acide chondroïtine sulfurique, l'acide hyaluronique de haut ou de bas poids moléculaire ou encore l'acide hyaluronique potentialisé par un dérivé de silanol tel que l'actif Epidermosil® commercialisé par la société Exymol, et l'acide mucoitinsulfurique; l'acide caronique; l'atelo collagène; le chlorestéryl-12-hydroxystéarate; les sels biliaires, une composante principale du FHN (facteur d'hydratation naturelle) comme un sel de l'acide pyrrolidone carboxylique et un sel d'acide lactique, un analogue d'acide aminé tel que l'urée, la cystéine et la sérine; un collagène soluble à chaîne courte, les PPG diglycérine, les homo- et copolymères de 2-méthacryloyloxyéthylphosphorylcholine comme le Lipidure HM et le Lipidure PBM de NOF; l'allantoïne; des dérivés de glycérine tels que le PEG / PPG / polybutylène Glycol-8/5/3 Glycérine de NOF vendu sous la dénomination commerciale Wilbride®S753 ou encore le glycerylpolymethacrylate de Sederma vendu sous la dénomination commerciale Lubragel®MS; la triméthylglycine vendu sous la dénomination commerciale Aminocoat® par la société Ashahi Kasei Chemicals et divers extraits de plantes tels que des extraits de Castanea sativa, des protéines de noisette hydrolysées, les polysaccharides de Tuberosa Polyanthes, l'huile de noyau d'Argania spinosa et les extraits de nacre contenant un conchyoline qui sont vendus notamment par la compagnie Maruzen (Japon) sous le nom commercial Pearl Extract®.

D'autres exemples d'agents hydratants incluent les composés stimulant l'expression de la matriptase MT/SP1, tel qu'un extrait de pulpe de caroube, ainsi que les agents stimulant l'expression de CERT, d'ARNT2 ou de FN3K ou FN3K RP ; les agents augmentant la prolifération ou la différenciation des kératinocytes, soit directement, soit indirectement en stimulant par exemple la production de β-endorphines, tels que les extraits de *Thermus thermophilus* ou de coques de fèves de *Theobroma cacao,* les extraits hydrosolubles de maïs, les extraits peptidiques de *Voandzeia subterranea* et le niacinamide ; les lipides épidermiques et les agents augmentant la synthèse de lipides épidermiques, soit directement, soit en stimulant certaines β-glucosidases qui modulent la déglycosylation de précurseurs lipidiques comme le glucosylcéramide en céramides, tels que les phospholipides, les céramides, les hydrolysats de protéine de lupin et les dérivés d'acide dihydrojasmonique.

Des exemples d'agents anti-pollution incluent l'extrait de graines de Moringa pterygosperma (par exemple le Purisoft® de LSN); l'extrait de beurre de karité (par exemple Detoxyl® de Silab), un mélange d'extrait de lierre, d'acide phytique, d'extrait de graine de tournesol (par exemple l'Osmopur® de Sederma).

Des exemples d'agents kératolytiques incluent les α-hydroxyacides (par exemple les acides glycolique, lactique, citrique, malique, mandélique, ou tartrique) et les β-hydroxyacides (par exemple l'acide salicylique), et leurs esters, tels que les C12-13 alkyl lactates, et les extraits de plantes contenant ces hydroxyacides, tels que des extraits d'Hibiscus sabdriffa.

Des exemples d'astringents incluent les extraits d'hamamélis.

Des exemples d'agents anti-inflammatoires incluent le bisabolol, l'allantoïne, l'acide tranexamique, l'oxyde de zinc, l'oxyde de soufre et ses dérivés, le sulfate de chondroïtine, l'acide glycyrrhizinique et ses dérivés tels que les glycyrrhizinates.

Des exemples d'agents blanchissants incluent l'arbutine et ses dérivés, l'acide férulique (tel que le Cytovector® : eau, glycol, lécithine, acide férulique, hydroxyéthylcellulose, commercialisé par BASF) et ses dérivés, l'acide kojique, le résorcinol, l'acide lipoïque et ses dérivés tel que le monolipoate de resvératrol diacétate tel que décrit dans la demande de brevet WO2006134282, l'acide ellagique, le leucodopachrome et ses dérivés, la vitamine B3, l'acide linoléique et ses dérivés, les céramides et leurs homologues, un peptide tel que décrit dans la demande de brevet WO2009010356, un bioprécurseur tel que décrit dans la demande de brevet WO2006134282 ou un sel de tranexamate tel que le sel de chlorhydrate de tranexamate cétylique, un extrait de réglisse (extrait de Glycyrrhiza glabra), qui est vendu notamment par la société Maruzen sous le nom commercial Licorice extract®, un agent blanchissant ayant également un effet antioxydant, comme les composés de vitamine C, y compris les sels d'ascorbate, les esters ascorbyle d'acides gras ou d'acide sorbique, et d'autres dérivés de l'acide ascorbique, par exemple, les phosphates d'ascorbyle, tels que le magnésium ascorbyl phosphate et le sodium ascorbyl phosphate, ou les esters de saccharide d'acide ascorbique, qui incluent, par exemple, l'ascorbyle-2-glucoside, le L-ascorbate de 2-0-alpha-D-glucopyranosyle, ou le L-ascorbate de 6-0-bêta-D-galactopyranosyle. Un agent actif de ce type est vendu en particulier par la société DKSH sous le nom commercial Ascorbyl glucoside®.

Des exemples d'agents favorisant la microcirculation incluent un extrait de lupin (tel que l'Eclaline® de Silab), de ruscus, de marron d'inde, de lierre, de ginseng ou de mélilot, la caféine, le nicotinate et ses dérivés, un extrait d'algue de Corallina officinalis tel que celui commercialisé par CODIF ; et leurs mélanges. Ces agents actifs sur la microcirculation cutanée peuvent être utilisés pour éviter le ternissement du teint et/ou améliorer l'homogénéisation et l'éclat du teint.

La composition utilisée selon l'invention peut en outre comprendre en plus d'un extrait de fleur de *Camellia japonica,* au moins un actif choisi parmi : les agents stimulant l'expression de la tensine 1 tel qu'un extrait d'élémi; les agents stimulant l'expression de la FN3K et/ou de la FN3K RP tel que un extrait de *Butea frondosa* ; les agents stimulant l'expression de CERT ou d'ARNT2; les agents stimulant la production de facteurs de croissance; les agents anti-glycation ou déglycants; les agents augmentant la synthèse de collagène ou prévenant sa dégradation (agents anti-collagénases, notamment inhibiteurs de métalloprotéinases matricielles) en particulier les agents augmentant la synthèse de collagène IV et/ou de hyaluronane et/ou de fibronectine, tel qu'au moins un oligopeptide acylé, notamment celui commercialisé par la société SEDERMA sous la dénomination commerciale Matrixyl® 3000; les agents augmentant la synthèse d'élastine ou prévenant sa dégradation (agents anti-élastases); les agents augmentant la synthèse de glycosaminoglycanes ou de protéoglycanes ou prévenant leur dégradation (agents anti-protéoglycanases) tel que l'actif Epidermosil® (acide hyaluronique associé au méthylsilanetriol) commercialisé par la société Exsymol; les agents stimulant la synthèse d'intégrines par les fibroblastes ; les agents augmentant la prolifération des fibloblastes; les agents facilitant l'absorption percutanée tels que les alcools, les alcools gras et les acides gras et leurs dérivés ester ou éther, les pyrrolidones, les 4-alkyl-oxazolidin-2-ones telle que la 4-decyloxazolidin-2-one ; les terpènes, les huiles essentielles et les α-hydroxyacides; et leurs mélanges, sans que cette liste ne soit limitative.

Il est apparu à la Demanderesse que l'association d'au moins un extrait de fleur de *Camellia japonica* avec un ou plusieurs des actifs décrits précédemment permettait de combiner avantageusement dans une même formule les effets de ces combinaisons d'actifs et d'obtenir ainsi une hydratation maximale et de longue durée de la peau.

La composition cosmétique selon l'invention renferme donc avantageusement au moins un extrait de fleur de *Camellia japonica* et au moins un actif choisi parmi les agents hydratants.

Plus particulièrement, elle peut contenir au moins un actif choisi parmi : un extrait fermenté de Thermus thermophilus ; un extrait de racine de Zingiber officinale (Gingembre) ; l'acide hyaluronique et ses dérivés ; un extrait de pulpe de caroube ; et leurs mélanges.

L'invention a également pour objet l'utilisation cosmétique de l'extrait de fleur de Camellia *japonica* tel que décrit précédemment ou de la composition cosmétique précédemment décrite pour hydrater et/ou protéger la peau humaine vis-à-vis du dessèchement.

Dans ce mode de réalisation, l'extrait ou la composition est appliqué sur une peau sèche non pathologique.

L'invention sera maintenant illustrée par les exemples non limitatifs suivants.

### EXEMPLE 1 - Préparation d'un extrait huileux de fleurs de Camellia japonica Alba plena

Un extrait huileux est préparé à partir de 2 kg de fleurs blanches de *Camellia japonica Alba plena* et de 9 kg d'huile raffinée de camélia.

Les fleurs fraichement isolées sont déshydratées jusqu'à une teneur en matière sèche de 90 % puis refroidies à -80°C pour être immédiatement broyées dans un broyeur à hélices, à une température comprise entre - 30 et -80°C. On obtient une poudre fine de granulométrie comprise entre 100 et 300 µm et régulière.

On met ensuite en œuvre le procédé comprenant les étapes suivantes :
a) La poudre de fleurs et l'huile raffinée de camélia sont introduites dans un réacteur en acier inoxydable fermé, qui est placé sous atmosphère saturée d'azote. Le mélange est maintenu à la température ambiante pendant 24 h heures environ, de sorte à assurer l'imprégnation de la poudre végétale par l'huile.
b) Le mélange est placé dans un réacteur fermé équipé d'un générateur d'ultrasons, sous atmosphère saturée d'azote, et soumis à traitement par ultrasons à une fréquence de 20 kHz, à température ambiante, pendant 15 minutes.
c) Le mélange est placé dans un réacteur fermé équipé d'un agitateur et sous atmosphère saturée d'azote, puis soumis à un traitement par micro-ondes, sous une puissance de 0,4 kW / kg de mélange pendant une durée de 7 min. La température maximale atteinte est de 118°C.

Les étapes b) et c) sont répétées deux fois successivement. Entre chaque étape, il est réalisé un refroidissement du mélange à température ambiante pendant 3 h sous atmosphère saturée d'azote.

Le mélange ainsi obtenu est soumis à centrifugation à 5 000 tours/min pendant 15 minutes, à une température de 40°C, sur une toile filtrante de porosité de 5 pm, afin de séparer la fraction huileuse (extrait huileux) des matières solides (tourteau gras) et d'obtenir un extrait liquide et homogène.

Après retour à température ambiante, l'extrait huileux obtenu se présente sous forme d'une huile fluide, homogène de couleur jaune clair et d'odeur florale peu intense. Dans chaque cas, l'extrait huileux obtenu présente une teneur en eau inférieure à 0,2 g/100 g, une acidité oléique inférieure ou égale à 0,4 % et un indice de peroxyde inférieur à 3 méq O_{2/}kg.

### EXEMPLE 2 - Test de stimulation de l'expression du gène KRT2 sur des kératinocytes traités par un extrait de fleur de Camellia japonica obtenu à l'exemple 1

### Protocole :

Les effets de l'extrait de l'Exemple 1 ou de l'huile de Camélia raffinée commercialisée par la société ARDEX ont été évalués par PCR en temps réel (en anglais RT-PCR pour « real-time polymerase chain reaction »), en vue de quantifier l'expression du gène kératine 2 (KRT2) dans un échantillon traité par rapport à un échantillon témoin non traité.

Les résultats sont exprimés en pourcentage d'augmentation ou de diminution de l'expression du gène cible (KRT2) dans l'échantillon traité. Les résultats sont normalisés par rapport à l'expression du même gène dans l'échantillon témoin non traité, qui est fixée à 100%.

Précisément, le test a été réalisé sur des kératinocytes humains normaux cultivés jusqu'à post-confluence puis traités ou non pendant 24 heures, en triplicat. Les kératinocytes proviennent de deux donneurs indépendants.

L'ARNm a été isolé en utilisant le kit RNeasy (QIAGEN) et quantifié en utilisant le kit QuantIT Ribogreen (Invitrogen) selon les recommandations du fabricant. La transcription inverse en ADNc a été effectuée à l'aide du kit iScript Reverse Transcription SuperMix (Biorad) également selon les recommandations du fabricant.

La mesure quantitative de PCR en temps réel a été effectuée en utilisant l'appareil iCYCLER IQ (Biorad), les sondes Taqman spécifiques des gènes KRT2 et béta-2-microglobuline (gène de référence) (Applied Biosystems) et le IQ Supermix (BioRad).

### Résultats :

**Tableau 1**

| | Concentration⁽¹⁾ | Stimulation de l'expression de KRT2(%) |
|---|---|---|
| Kératinocytes non traités | - | 100 |
| Extrait de fleur de Camellia *japonica* selon l'exemple 1 | 0,025% | 170 |
| Huile de Camélia raffinée commercialisée par la société ARDEX | 0,025% | 93 |

| | | |
|---|---|---|
| (1) les concentrations des extraits sont exprimées en poids d'extrait brut par poids de préparation (l'extrait obtenu selon l'exemple 1 étant dilué dans le milieu de culture pour la croissance de kératinocytes) | | |

L'extrait de fleur de Camellia *japonica* stimule l'expression de l'ARNm de KRT2 par rapport au témoin non traité et par rapport au comparatif traité par une huile de Camellia raffinée obtenue par un autre procédé (i.e. celui de la société ARDEX) que celui de la présente demande.

### Exemple 3 : Composition cosmétique (sérum H/E)

La composition suivante peut être préparée de façon classique pour l'homme du métier. Les quantités indiquées ci-dessous sont exprimées en pourcentages pondéraux. Les ingrédients en majuscules sont identifiés conformément à la dénomination INCI.

| **Nom INCI** | **% (poids/poids)** |
|---|---|
| WATER | QSP |
| | 100,00 |
| Agent chélatant | 0,05 |
| Ajusteur de pH | 0,05 |
| Conservateur | 0,05 |
| Glycol | 3,25 |
| AMMONIUM ACRYLOYLDIMETHYLTAURATE/VP COPOLYMER | 1,20 |
| ACRYLATES/C10-30 ALKYL ACRYLATE CROSSPOLYMER | 0,20 |
| GLYCERIN | 3,00 |
| GLYCERYLPOLYMETHACRYLATE | 4.18 |
| SODIUM ACETYLATED HYALURONATE | 0,05 |
| PEG/PPG/POLYBUTYLENE GLYCOL-8/5/3 GLYCERIN | 2,00 |
| SODIUM PCA | 3,00 |
| Huile | 10,00 |
| ALCOHOL | 8,00 |
| FRAGRANCE | 0,30 |
| ZINGIBER OFFICINALE (GINGER) ROOT EXTRACT ¹ | 0,10 |
| HYALURONIC ACID & SILANETRIOL & CITRIC ACID 2 | 5,00 |
| OLEOACTIF CAMELIA BLANC ³ | 0,10 |
| CROSSLINKED POLYMETHYLMETHACRYLATE ⁴ | 0, 75 |
| HDI / TRIMETHYLOL HEXYLLACTONE CROSSPOLYMER & SILICA ⁵ | 0, 75 |
| Extrait de pulpe de caroube | 0,10 |

| | |
|---|---|
| ⁽¹⁾ Blue Malagasy Ginger® vendu par la société BIOLANDES ⁽²⁾ EPIDERMOSIL® vendu par la société EXSYMOL ⁽³⁾ tel que décrit à l'Exemple 1 ⁽⁴⁾ MICROPEARL® M310 de SEPPIC ⁽⁵⁾ PLASTIC POWDER® D-400 / BPD-500® de KOBO | |

Cette composition peut être appliquée quotidiennement, matin et/ou soir, sur des peaux particulièrement déshydratées et/ou exposées aux agressions de l'environnement, pour améliorer leur confort et uniformiser le teint.

### Exemple 4 : Composition cosmétique (sérum H/E)

La composition suivante peut être préparée de façon classique pour l'homme du métier. Les quantités indiquées ci-dessous sont exprimées en pourcentages pondéraux. Les ingrédients en majuscules sont identifiés conformément à la dénomination INCI.

| **Nom INCI** | **% (poids/poids)** |
|---|---|
| WATER | QSP |
| | 100,00 |
| Agent chélatant | 0,05 |
| Ajusteur de pH | 0,05 |
| Conservateur | 0,05 |
| Glycol | 15,00 |
| CARBOMER | 0,08 |
| ACRYLATES/C10-30 ALKYL ACRYLATE CROSSPOLYMER | 0,17 |
| GLYCERIN | 15,00 |
| DIPENTAERYTHRITYL HEXACAPRYLATE / HEXACAPRATE | 4.3 |
| ISONONYL ISONONANOATE | 4.3 |
| FRAGRANCE | 0.30 |
| PVP | 0.095 |
| AMODIMETHICONE | 0.04 |
| ZINGIBER OFFICINALE (GINGER) ROOT EXTRACT¹ | 0.10 |
| OLEOACTIF CAMELIA BLANC² | **0.1** |
| SODIUM HYALURONATE | 0.01 |

| | |
|---|---|
| ⁽¹⁾ Blue Malagasy Ginger® vendu par la société BIOLANDES ⁽²⁾ tel que décrit à l'Exemple 1 | |

Cette composition peut être appliquée quotidiennement, matin et/ou soir, sur des peaux particulièrement déshydratées et/ou exposées aux agressions de l'environnement, pour améliorer leur confort et uniformiser le teint.

## Revendications

1. Extrait huileux de fleur de Camellia *japonica* susceptible d'être obtenu au moyen d'un procédé d'extraction comprenant les étapes suivantes :
a) le mélange et l'imprégnation d'une poudre de fleurs de Camellia *japonica* avec un corps gras ou un mélange de corps gras à une température supérieure à la température de fusion dudit corps gras ou dudit mélange et sous atmosphère dépourvue ou essentiellement dépourvue d'oxygène,
b) la micro-dispersion de la poudre de fleurs de Camellia *japonica* dans le corps gras ou ledit mélange de corps gras à une température supérieure à la température de fusion dudit corps gras ou dudit mélange, sous atmosphère dépourvue ou essentiellement dépourvue d'oxygène,
c) le chauffage du mélange ainsi obtenu à une température comprise entre 60 et 180°C pendant une durée comprise entre 1 et 10 minutes, sous atmosphère dépourvue ou essentiellement dépourvue d'oxygène,
l'étape c) pouvant être réalisée avant, pendant ou après l'étape b).

2. Extrait selon la revendication 1, **caractérisé en ce que** la fleur de Camellia provient de la variété Camellia *japonica* Alba Plena.

3. Extrait selon l'une quelconque des revendications précédentes, **caractérisé en ce que** le ratio entre la poudre de fleurs de Camellia *japonica* et le corps gras dans l'étape a) est compris entre 1:0,5 et 1:20, de préférence entre 1:19 et 1:1, et plus préférentiellement entre 1:9 et 1:3, ratio exprimé en masse/masse de corps gras ou en masse/volume de corps gras.

4. Extrait selon l'une quelconque des revendications précédentes, **caractérisé en ce que** le ou les corps gras est une huile végétale liquide à température ambiante, un beurre végétal présentant un point de fusion compris entre 25 et 40°C, ou une cire végétale présentant un point de fusion supérieur à 40°C, et de préférence, le ou les corps gras est une huile choisie parmi l'huile de camélia, l'huile de colza, l'huile de tournesol, l'huile d'olive, l'huile de sésame, l'huile de noyaux d'abricot, l'huile de pépin de raisin, l'huile d'amande douce, l'huile de carthame, l'huile de noisette, l'huile d'argan, l'huile de rosier muscat, l'huile d'onagre, l'huile de bourrache, la cire liquide de jojoba, et leurs mélanges.

5. Extrait selon l'une quelconque des revendications précédentes, **caractérisé en ce que** l'étape a) est conduite à une température supérieure au point de fusion du corps gras ou du mélange de corps gras utilisé, de préférence la température est comprise entre cette température de fusion et la température de fusion +20°C, de préférence +10°C, pendant une durée comprise entre 1 et 48 heures, de préférence entre 5 et 40 heures, plus préférentiellement entre 12 et 36 heures, encore plus préférentiellement entre 20 et 30 heures, et selon un mode particulièrement préféré de réalisation, environ 24 heures.

6. Extrait selon l'une quelconque des revendications précédentes, **caractérisé en ce que** l'étape b) comprend un traitement par cavitation ultrasonore, pendant une durée comprise entre 2 et 30 minutes, de préférence entre 10 et 20 minutes, à une fréquence de cavitation inférieure à 100 kHz, de préférence comprise entre 20 et 30 kHz.

7. Extrait selon l'une quelconque des revendications précédentes, **caractérisé en ce que** l'étape b) est conduite à température ambiante ou à une température supérieure au point de fusion du corps gras ou mélange de corps gras utilisé, de préférence la température est comprise entre la température de fusion et la température de fusion +20°C, de préférence +10°C.

8. Extrait selon l'une quelconque des revendications précédentes, **caractérisé en ce que** l'étape c) est effectuée à une température comprise entre 100 et 150°C, de préférence entre 110 et 130°C, pendant une durée allant de 1 à 10 minutes, de préférence de 1 à 5 minutes, et plus préférentiellement de 1 à 3 minutes.

9. Extrait selon la revendication précédente, **caractérisé en ce que** la température de l'étape c) est obtenue par un traitement micro-ondes de puissance utile allant de 500 à 10000 Watts par kilogramme de mélange, de préférence de l'ordre de 700 à 1500 Watts par kilogramme de mélange, et plus préférentiellement de l'ordre de 1000 Watts par kilogramme de mélange.

10. Extrait selon l'une quelconque des revendications précédentes, **caractérisé en ce que** la poudre de fleurs de Camellia *japonica* est apportée sous la forme d'un produit dispersible obtenu par broyage à une température comprise entre -20 et -80°C.

11. Extrait selon l'une quelconque des revendications précédentes, **caractérisé en ce que** les étapes a), b) et/ou c) sont avantageusement conduites en l'absence de lumière ou de tout rayonnement oxydant tels que les UV.

12. Extrait selon l'une quelconque des revendications précédentes, **caractérisé en ce que** les étapes b) et c) réalisées au moins une seconde fois et de préférence en n fois ; elles correspondent alors aux étapes bn) et cn), n correspondant au nombre total de répétition du cycle {(étape a) + (étape b)}, n étant au moins égal à 2, de préférence n étant égal à 2.

13. Composition cosmétique renfermant, dans un milieu physiologiquement acceptable, au moins un extrait de fleur de Camellia *japonica* selon l'une quelconque des revendications 1 à 12.

14. Composition selon la revendication 13, **caractérisée en ce qu'**elle renferme en outre au moins un actif choisi parmi les agents hydratants, les antioxydants, les agents favorisant la microcirculation, et leurs mélanges.

15. Composition selon la revendication 14, **caractérisée en ce que** l'actif est choisi parmi : un extrait fermenté de Thermus thermophilus ; un extrait de racine de Zingiber officinale ; l'acide hyaluronique et ses dérivés ; un extrait de pulpe de caroube ; et leurs mélanges.

16. Utilisation cosmétique de l'extrait de fleur de Camellia *japonica* selon l'une quelconque des revendications 1 à 12 ou de la composition selon l'une quelconque des revendications 13 à 15 pour hydrater et/ou protéger la peau humaine vis-à-vis du dessèchement.

17. Utilisation selon la revendication 16, **caractérisée en ce que** l'extrait ou la composition est appliqué sur une peau sèche non pathologique.

## Patentansprüche

1. Öliger Blumenextrakt von Camellia *japonica,* erhältlich durch ein Extraktionsverfahren, umfassend die folgenden Schritte:
a) Mischen und Imprägnieren eines Blumenpulvers von Camellia *japonica* mit einem Fettstoff oder einer Mischung von Fettstoffen bei einer Temperatur über der Schmelztemperatur des Fettstoffs oder der Mischung und in einer Atmosphäre, die frei oder im Wesentlichen frei von Sauerstoff ist,
b) Mikrodispergieren des Blumenpulvers von Camellia *japonica* in dem Fettstoff oder der Mischung von Fettstoffen bei einer Temperatur über der Schmelztemperatur des Fettstoffs oder der Mischung in einer Atmosphäre, die frei oder im Wesentlichen frei von Sauerstoff ist,
c) Erhitzen der so erhaltenen Mischung bei einer/auf eine Temperatur zwischen 60 und 180 °C für einen Zeitraum zwischen 1 und 10 Minuten, in einer Atmosphäre die frei oder im Wesentlichen frei von Sauerstoff ist,
wobei der Schritt c) vor, während oder nach dem Schritt b) durchgeführt werden kann.

2. Extrakt nach Anspruch 1, **dadurch gekennzeichnet, dass** die Camellia Blume von der Sorte Camellia *japonica* Alba Plena stammt.

3. Extrakt nach einem der vorhergehenden Ansprüche, **dadurch gekennzeichnet, dass** das Verhältnis zwischen dem Blumenpulver von Camellia *japonica* und dem Fettstoff in Schritt a) zwischen 1:0,5 und 1:20, vorzugsweise zwischen 1:19 und 1:1, und stärker bevorzugt zwischen 1:9 und 1:3, liegt, wobei das Verhältnis ausgedrückt ist als Masse/Masse an Fettstoff oder als Masse/Volumen an Fettstoff.

4. Extrakt nach einem der vorhergehenden Ansprüche, **dadurch gekennzeichnet, dass** der Fettstoff oder die Fettstoffe ein bei Raumtemperatur flüssiges Pflanzenöl, eine Pflanzenbutter mit einem Schmelzpunkt zwischen 25 und 40 °C oder ein Pflanzenwachs mit einem Schmelzpunkt über 40 °C ist/sind, und bevorzugt, dass der Fettstoff oder die Fettstoffe ein Öl ist/sind, ausgewählt aus Kamelienöl, Rapsöl, Sonnenblumenöl, Olivenöl, Sesamöl, Aprikosenkernöl, Traubenkernöl, Süßmandelöl, Färberdistelöl, Haselnussöl, Arganöl, Wildrosenöl, Nachtkerzenöl, Borretschöl, flüssigem Jojobawachs und Mischungen davon.

5. Extrakt nach einem der vorhergehenden Ansprüche, **dadurch gekennzeichnet, dass** der Schritt a) bei einer Temperatur über dem Schmelzpunkt des eingesetzten Fettstoffes oder der eingesetzten Fettstoffmischung durchgeführt wird, vorzugsweise dass die Temperatur zwischen dieser Schmelztemperatur und der Schmelztemperatur + 20 °C, bevorzugt + 10 °C, liegt, für einen Zeitraum, der zwischen 1 und 48 Stunden, bevorzugt zwischen 5 und 40 Stunden, stärker bevorzugt zwischen 12 und 36 Stunden, noch stärker bevorzugt zwischen 20 und 30 Stunden und gemäß einer besonders bevorzugten Ausführungsform, bei etwa 24 Stunden liegt.

6. Extrakt nach einem der vorhergehenden Ansprüche, **dadurch gekennzeichnet, dass** der Schritt b) eine Behandlung durch Ultraschallkavitation für einen Zeitraum, der zwischen 2 und 30 Minuten, vorzugsweise zwischen 10 und 20 Minuten, liegt, bei einer Kavitationsfrequenz, die niedriger als 100 kHz, vorzugsweise zwischen 20 und 30 kHz ist, umfasst.

7. Extrakt nach einem der vorhergehenden Ansprüche, **dadurch gekennzeichnet, dass** der Schritt b) bei Umgebungstemperatur oder bei einer Temperatur oberhalb des Schmelzpunktes des verwendeten Fettstoffs oder der verwendeten Fettstoffmischung durchgeführt wird, wobei die Temperatur vorzugsweise zwischen der Schmelztemperatur und der Schmelztemperatur + 20 °C, vorzugsweise + 10 °C, liegt.

8. Extrakt nach einem der vorhergehenden Ansprüche, **dadurch gekennzeichnet, dass** der Schritt c) bei einer Temperatur zwischen 100 und 150 °C, vorzugsweise zwischen 110 und 130 °C, für eine Dauer von 1 bis 10 Minuten, bevorzugt von 1 bis 5 Minuten und stärker bevorzugt von 1 bis 3 Minuten, durchgeführt wird.

9. Extrakt nach dem vorhergehenden Anspruch, **dadurch gekennzeichnet, dass** die Temperatur von Schritt c) durch eine Mikrowellenbehandlung mit einer Nutzleistung von 500 bis 10.000 Watt pro Kilogramm Mischung, vorzugsweise in der Größenordnung von 700 bis 1.500 Watt pro Kilogramm Mischung und stärker bevorzugt in der Größenordnung von 1.000 Watt pro Kilogramm Mischung erhalten wird.

10. Extrakt nach einem der vorhergehenden Ansprüche, **dadurch gekennzeichnet, dass** das Blumenpulver von Camellia *japonica* in Form eines dispergierbaren Produkts, das durch Zerkleinern bei einer Temperatur zwischen -20 und -80 °C erhalten wird, bereitgestellt ist.

11. Extrakt nach einem der vorhergehenden Ansprüche, **dadurch gekennzeichnet, dass** die Schritte a), b) und/oder c) vorteilhafterweise in der Abwesenheit von Licht oder jeglicher oxidierender Strahlung wie UV durchgeführt wird/werden.

12. Extrakt nach einem der vorhergehenden Ansprüche, **dadurch gekennzeichnet, dass** die Schritte b) und c) mindestens ein zweites Mal und vorzugsweise n-mal durchgeführt werden; wobei sie dann den Schritten bn) und cn) entsprechen, wobei n der Gesamtanzahl der Wiederholungen des Zyklus {(Schritt a) + (Schritt b)} entspricht, wobei n mindestens gleich 2 ist, vorzugsweise wobei n gleich 2 ist.

13. Kosmetische Zusammensetzung, die in einem physiologisch akzeptablen Medium mindestens einen Blumenextrakt von Camellia *japonica* nach einem der Ansprüche 1 bis 12 umfasst.

14. Zusammensetzung nach Anspruch 13, **dadurch gekennzeichnet, dass** sie ferner mindestens einen Wirkstoff umfasst, ausgewählt aus Hydratisierungsmitteln, Antioxidationsmitteln, Mitteln zur Förderung der Mikrozirkulation und Mischungen davon.

15. Zusammensetzung nach Anspruch 14, **dadurch gekennzeichnet, dass** der Wirkstoff ausgewählt ist aus: einem fermentierten Extrakt von Thermus thermophilus; einem Wurzelextrakt von Zingiber officinale; Hyaluronsäure und ihren Derivaten; einem Extrakt von Johannisbrotfruchtfleisch; und Mischungen davon.

16. Kosmetische Verwendung des Blumenextrakts von Camellia *japonica* nach einem der Ansprüche 1 bis 12 oder der Zusammensetzung nach einem der Ansprüche 13 bis 15 zur Hydratisierung und/oder zum Schutz der menschlichen Haut gegen die Austrocknung.

17. Verwendung nach Anspruch 16, **dadurch gekennzeichnet, dass** der Extrakt oder die Zusammensetzung auf nicht pathologisch trockene Haut aufgetragen wird.

## Claims

1. An oily extract of *Camellia japonica* flower which can be obtained by means of an extraction process comprising the following steps:
a) mixing and impregnation of a *Camellia japonica* flower powder with a fatty substance or a fatty substance mixture at a temperature above the melting point of said fatty substance or of said mixture and under an atmosphere free or essentially free of oxygen,
b) microdispersion of the *Camellia japonica* flower powder in the fatty substance or said fatty substance mixture at a temperature above the melting point of said fatty substance or of said mixture, under an atmosphere free or essentially free of oxygen,
c) heating of the mixture thus obtained at a temperature of between 60 and 180°C for a period of between 1 and 10 minutes, under an atmosphere free or essentially free of oxygen,
it being possible for step c) to be carried out before, during or after step b).

2. The extract as claimed in claim 1, **characterized in that** the camellia flower comes from the variety *Camellia japonica Alba plena.*

3. The extract as claimed in either one of the preceding claims, **characterized in that** the ratio between the *Camellia japonica* flower powder and the fatty substance in step a) is between 1:0.5 and 1:20, preferably between 1:19 and 1:1, and more preferentially between 1:9 and 1:3, said ratio being expressed by weight/weight of fatty substance or by weight/volume of fatty substance.

4. The extract as claimed in any one of the preceding claims, **characterized in that** the fatty substance (s) is a plant oil that is liquid at ambient temperature, a plant butter having a melting point of between 25 and 40°C, or a plant wax having a melting point above 40°C, and preferably the fatty substance(s) is an oil chosen from camellia oil, rapeseed oil, sunflower oil, olive oil, sesame oil, apricot kernel oil, grape seed oil, sweet almond oil, safflower oil, hazelnut oil, argon oil, rose musk oil, evening primrose oil, borage oil, jojoba liquid wax, and mixtures thereof.

5. The extract as claimed in any one of the preceding claims, **characterized in that** step a) is carried out at a temperature above the melting point of the fatty substance or of the fatty substance mixture used, preferably the temperature is between this melting point and the melting point + 20°C, preferably + 10°C, for a period of between 1 and 48 hours, preferably between 5 and 40 hours, more preferentially between 12 and 36 hours, even more preferentially between 20 and 30 hours, and, according to one particularly preferred embodiment, approximately 24 hours.

6. The extract as claimed in any one of the preceding claims, **characterized in that** step b) comprises a treatment by ultrasonic cavitation, for a period of between 2 and 30 minutes, preferably between 10 and 20 minutes, at a cavitation frequency of less than 100 kHz, preferably of between 20 and 30 kHz.

7. The extract as claimed in any one of the preceding claims, **characterized in that** step b) is carried out at ambient temperature or at a temperature above the melting point of the fatty substance or fatty substance mixture used, preferably the temperature is between the melting point and the melting point +20°C, preferably +10°C.

8. The extract as claimed in any one of the preceding claims, **characterized in that** step c) is carried out at a temperature of between 100 and 150°C, preferably between 110 and 130°C, for a period ranging from 1 to 10 minutes, preferably from 1 to 5 minutes, and more preferentially from 1 to 3 minutes.

9. The extract as claimed in the preceding claim, **characterized in that** the temperature of step c) is obtained by means of a microwave treatment having a working power ranging from 500 to 10 000 watts per kilogram of mixture, preferably of about 700 to 1500 watts per kilogram of mixture, and more preferentially of about 1000 watts per kilogram of mixture.

10. The extract as claimed in any one of the preceding claims, **characterized in that** the *Camellia japonica* flower powder is provided in the form of a dispersible product obtained by milling at a temperature between -20 and -80°C.

11. The extract as claimed in any one of the preceding claims, **characterized in that** steps a), b) and/or c) are advantageously carried out in the absence of light or of any oxidizing radiation such as UV radiation.

12. The extract as claimed in any one of the preceding claims, **characterized in that** steps b) and c) are carried out at least a second time and preferably n times; they then correspond to steps bn) and cn), n corresponding to the total number of repetitions of the cycle {(step a) + (step b)}, n being at least equal to 2, preferably n being equal to 2.

13. A cosmetic composition containing, in a physiologically acceptable medium, at least one *Camellia japonica* flower extract as claimed in any one of claims 1 to 12.

14. The composition as claimed in claim 13, **characterized in that** it also contains at least one active agent chosen from moisturizing agents, antioxidants, agents which promote the microcirculation, and mixtures thereof.

15. The composition as claimed in claim 14, **characterized in that** the active agent is chosen from: a fermented extract of Thermus thermophilus; an extract of Zingiber officinale root; hyaluronic acid and derivatives thereof; an extract of locust bean pulp; and mixtures thereof.

16. The cosmetic use of the *Camellia japonica* flower extract as claimed in any one of claims 1 to 12 or of the composition as claimed in any one of claims 13 to 15, for moisturizing and/or protecting human skin against drying out.

17. The use as claimed in claim 16, **characterized in that** the extract or the composition is applied to nonpathological dry skin.
